# EUROPÄISCHE PATENTSCHRIFT

(11) **EP 1 772 158 B1**
(45) Veröffentlichungstag und Bekanntmachung des Hinweises auf die Patenterteilung: **18.11.2009**
(21) Anmeldenummer: 06019728.2
(22) Anmeldetag: 21.09.2006
(51) Int. Cl.: A61L 9/01, B65D 90/44

(54) **Verfahren zum Reduzieren von übelriechenden Substanzen in Tankbehältern**
Process vor reducing bad smelling substances in tank vessels
Procédé du reduction des substances malodorantes dans citernes

(30) Priorität: 06.10.2005 DE 102005047889
(43) Veröffentlichungstag der Anmeldung: 11.04.2007
(73) Patentinhaber: Air & D- Sarl, 67190 Rosheim (FR)
(72) Erfinder: Wuest, Robert, 67190 Rosheim (FR); Kerszenbaum, Laurent, 06110 Le Cannet (FR)
(74) Vertreter: Wagner, Jutta

(56) Entgegenhaltungen:
- GB-A- 1 492 400
- US-A- 3 628 758
- US-A- 4 238 461
- US-A- 5 017 351

## Beschreibung

Die Erfindung betrifft ein Verfahren zum Reduzieren von übelriechenden Substanzen in Tankbehältern für Schweröl oder Bitumen, in denen über dem Flüssigkeitsspiegel sich ein Gasraum mit den übelriechenden Substanzen befindet, wobei in den Gasraum zur Inertisierung gegen Explosionen ein Inertgas eingeleitet wird, das am oberen Ende des Tankbehälters wieder austritt.

Schweröle und verflüssigtes Bitumen werden im allgemeinen in großen Tankbehältern, die bis zu 40 m hoch sein können, gelagert. Je nach Füllgrad des Tanks befindet sich über dem Flüssigkeitsspiegel ein mehr oder weniger großer Gasraum, in den hinein flüchtige Bestandteile aus der Flüssigkeit verdunsten. Diese sind teilweise brennbar, so dass Explosionsgefahr besteht. Um dieser zu begegnen, werden in den Gasraum hinein Inertgase eingeleitet, die am oberen Ende des Tankbehälters zusammen mit den flüchtigen Bestandteilen wieder abgeführt werden. Diese flüchtigen Bestandteile bestehen im wesentlichen aus Kohlenwasserstoffen und Schwefelverbindungen, die in der Umgebung des Behälters einen üblen, zum Erbrechen reizenden Geruch verbreiten. Dies ist besonders gravierend, wenn der Tank gefüllt wird und durch den dabei auftretenden erhöhten Druck die Entgasung durch das Abluftrohr beschleunigt wird. Es ist nun praktisch nicht möglich, die übelriechenden Substanzen auf konventionelle Weise durch Absaugen zu entfernen, da einige ihrer Bestandteile in klebriger Form kondensieren und so die Absaugrohre verstopfen würden.

In der US-A 3,628,758 ist ein Verfahren zur Inertisierung des Gasraums über einem Flüssigkeitsspiegel eines Kraftstoffbehälters beschrieben, bei dem in den Gasraum ein Inertgas eingeleitet wird.

In der US-A 5,017,351 ist die Entfernung von Schadstoffen aus einem Luftstrom von Umgebungsluft, z.B. in Bürogebäuden, beschrieben. In den Luftstrom werden aus einer Düse Tröpfchen einer wässrigen Suspension eingespritzt, die gegebenenfalls chemische Agenzien, z.B. Hypochlorite oder Wasserstoffperoxid enthalten können.

In der US-A 4,238, 461 ist die Entfernung von übel riechenden Bestandteilen aus Gasströmen beschrieben, wobei das Gas mit fein verteilten Wassertröpfchen behandelt wird, die als reaktive Agenzien Oxidationsmittel, wie z.B.
Natriumhypochlorit, enthalten.

Der Erfindung lag nun die Aufgabe zugrunde, die übelriechenden Substanzen in dem Gasraum zu entfernen oder wenigstens stark zu reduzieren, um dadurch die Geruchsbelästigung zu vermindern.

Diese Aufgabe wird erfindungsgemäß dadurch gelöst, dass zusammen mit dem Inertgas bestimmte aktive Agentien in Form von Aldehyden, Ketonen, Estern oder Alkoholen, natürlichen, öligen Essenzen oder parfümartigen Substanzen in den Gasraum eingeleitet werden, die mit den übelriechenden Substanzen reagieren bzw. diese maskieren und dadurch die Geruchbelästigung in der Umgebungsluft vermindern.

Im industriellen Bereich werden recht große Tankbehälter verwendet, die z.B. 500 bis 15.000 to Schweröl bzw. Bitumen enthalten. Bitumen liegt dabei als 80 bis 200 C heiße Flüssigkeit vor, wodurch natürlich die Verdunstung von flüchtigen Bestandteilen begünstigt wird.

Je nach Füll- bzw. Entleerungsgrad des Tankbehälters macht der Gasraum über dem Flüssigkeitsspiegel 10 bis 90 % des Gesamtvolumens aus. In diesen Gasraum hinein wird zur Inertisierung gegen Explosionen ein Inertgas eingeleitet, z.B.

Stickstoff oder Wasserdampf, wobei letzterer im allgemeinen unter einem Druck von 2 bis 20 bar, vorzugsweise 2 bis 10 steht und eine Temperatur von 140 bis 200 °C aufweist. Erfindungsgemäß werden mit dem Inertgasstrom aktive Agentien in den Gasraum eingeführt. Dies sind erfindungsgemäß höhere Aldehyde, Ketone, Ester oder Alkohole, z.B. C7- bis C12- Aldehyde, oder natürliche ölige Essenzen, wie Harzöle, ferner parfümartige Substanzen, wie Vanillin, Menthol oder Alpha-Pinen.

Die aktiven Substanzen werden als wässrige Emulsion in Form feiner Tropfchen oder als Gas in das Zuleitungsrohr mit dem Inertgasstrom eingepresst und mit diesem zusammen in den Gasraum eingeleitet. Durch geeignete Ventile und Bypass- Leitungen kann die Menge des in den Gasraum eingeleiteten Inertgases, welches die aktiven Komponenten enthält, gesteuert werden, so dass z.B. bei niedrigem Flüssigkeitsstand, und insbesondere beim Befüllen des Tanks, kontinuierlich relativ viel und bei hohem Flüssigkeitsstand und entsprechend kleinem Gasraum sequenzweise relativ wenig Inertgas eingeleitet wird.

Bei einer bevorzugten Ausführungsform der Erfindung wird aus einem Vorratsgefäß eine z.B. 10 bis 30 Gew.%ige wässrige Emulsion der aktiven Agentien von einer Dosierpumpe angesaugt und dort mit zugeführtem Wasser verdünnt. Das Gemisch, das dann z.B. 0,5 bis 20 Gew.% aktive Substanzen enthält, wird durch eine Hochdruckpumpe unter einen Druck von z.B. 25 bis 120 bar gesetzt und in das Rohr, in dem das Inertgas geführt wird, eingeleitet. Am Ende der Leitung befindet sich eine oder mehrere feine Düsen, durch die die Emulsion durchgepresst wird. Dabei wird die wässrige Emulsion der aktiven Agentien versprüht und auf eine Partikelgröße von 5 bis 25 µm mikronisiert oder auch verdampft und in dieser Form in den Gasraum eingebracht. Es ist natürlich möglich, die Leitung, in der die Emulsion geführt wird, mit mehreren Inertgasrohren zu verbinden, so dass mehrere Tankbehälter gleichzeitig mit aktiven Agentien behandelt werden können. Durch Ventile bzw. Bypass-Leitungen kann die Behandlungsintensität dabei unterschiedlich gesteuert werden.

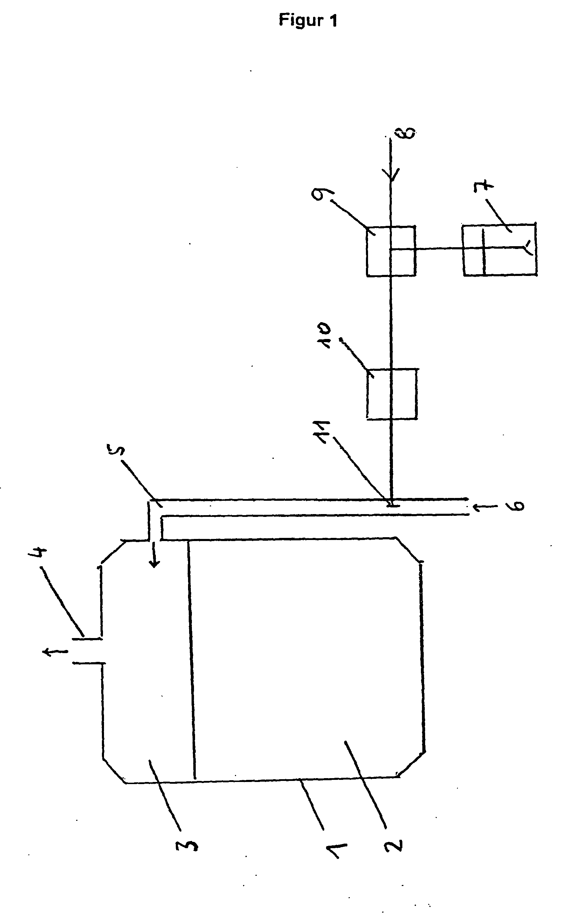

Die beigefügte Zeichnung zeigt eine schematische Verlaufsskizze der bevorzugten Ausführungsform. Dabei ist mit 1 der Tankbehälter bezeichnet, mit 2 das Schweröl bzw. das verflüssigte Bitumen, mit 3 der Gasraum über dem Flüssigkeitsspiegel, mit 4 der Austritt des Abgases, mit 5 Rohr, durch welches das Inertgas in den Gasraum eingeleitet wird, mit 6 die Inertgaszufuhr, mit 7 das Vorratsgefäß für die wässrige Emulsion der aktiven Agentien, mit 8 den Wasserstrom, der in die Dosierpumpe 9 eingeleitet wird, welche gleichzeitig die wässrige Emulsion aus dem Vorratsgefäß ansaugt, mit 10 die Hochdruckpumpe, in der das Gemisch aus Wasser und den aktiven Agentien unter Druck gesetzt wird und mit 11 die Düse, durch die das Gemisch in das Rohr 5 eingepresst und dabei mikronisiert wird.

Bei einer anderen Ausführungsform wird trockener, vorzugsweise kalter Dampf nach dem Venturi- Verfahren mit den aktiven Agentien vermischt, und die in öliger Form vorliegende Mischung wird in das Rohr mit dem Inertgas eingepresst und dabei zu Tröpfchen mit einer Partikelgröße von 0,02 bis 5 µm, vorzugsweise 0,08 bis 2 µm mikronisiert.

Bei einer weiteren Ausführungsform wird in einer Zweifluss- Düse einerseits Luft und andererseits eine wässrige Emulsion der aktiven Agentien eingeführt, in einer Mischkammerdüse vermischt, komprimiert und in das Rohr hinein ausgepresst und dabei auf eine Partikelgröße von 8 bis 40 µm mikronisiert.

Schließlich können die aktiven Agentien, die - wie in der EP-B 1 471 948 beschrieben - an einem gelförmigen Polymeren adsorbiert sind, durch darüber strömende Luft oder ein Inertgas wieder desorbiert werden und mit diesem zusammen in das Rohr mit dem Inertgasstrom eingeführt werden.

Die in den Gasraum eingeführten aktiven Agentien reagieren dort - oder kurz hinter dem Austritt des Inertgases aus dem Tankbehälter - mit den übelriechenden Substanzen und entfernen diese ganz oder zumindest weitgehend aus dem ausgetretenen Abgasstrom, so dass die Geruchsbelästigung weitgehend beseitigt ist. Parallel dazu können wohlriechende aktive Agentien auch die übelriechenden Substanzen maskieren und deren Geruch überdecken.

## Patentansprüche

1. Verfahren zum Reduzieren von übelriechenden Substanzen in Tankbehältern für Schweröl oder verflüssigtes Bitumen, in denen über dem Flüssigkeitsspiegel sich ein Gasraum mit den übelriechenden Substanzen befindet, wobei in den Gasraum zur Inertisierung gegen Explosionen ein Inertgas eingeleitet wird, das am oberen Ende des Tankbehälters wieder austritt, **dadurch gekennzeichnet, dass** zusammen mit dem Inertgas aktive Agentien in Form von Aldehyden, Ketonen, Estern und/oder Alkoholen oder natürlichen, öligen Essenzen oder parfümartigen Substanzen eingeleitet werden, die mit den übelriechenden Substanzen reagieren bzw. diese maskieren und dadurch die Geruchsbelästigung vermindern.

2. Verfahren nach Anspruch 1, **dadurch gekennzeichnet, dass** der Tankbehälter 500 bis 15.000 to Schweröl bzw. Bitumen enthält, wobei der Gasraum über dem Flüssigkeitsspiegel 10 bis 90 % des Gesamtvolumens ausmacht.

3. Verfahren nach Anspruch 1, **dadurch gekennzeichnet, dass** als Inertgas Stickstoff oder Wasserdampf eingeleitet wird.

4. Verfahren nach Anspruch 1, **dadurch gekennzeichnet, dass** die übelriechenden Substanzen Kohlenwasserstoffe und Schwefelverbindungen enthalten.

5. Verfahren nach Anspruch 1, **dadurch gekennzeichnet, dass** die aktiven Agentien Aldehyde, Ketone, Ester und/oder Alkohole sind.

6. Verfahren nach Anspruch 1, **dadurch gekennzeichnet, dass** die aktiven Agentien als wässrige Emulsion in Form feiner Tröpfchen mit einer Partikelgröße von weniger als 50 µm oder als Gas in den Inertgasstrom eingepresst und mit diesem zusammen in den Gasraum eingeführt werden, wo sie mit den übelriechenden Substanzen reagieren bzw. diese maskieren.

7. Verfahren nach Anspruch 6, **dadurch gekennzeichnet, dass** die aktiven Agentien als 0,5 bis 25 Gew.%ige wässrige Emulsion durch feine Düsen in Form feiner Tröpfchen mit einer Partikelgröße von 5 bis 25 µm in das Rohr eingepresst werden, in dem der Inertgasstrom geführt wird.

## Claims

1. Process for reducing foul-smelling substances in tank vessels for heavy oil or liquefied bitumen, in which a gas space harboring the foul-smelling substances is situated above the liquid level, whereby, for inerting against explosions, an inert gas is guided into the gas space and exits again at the upper end of the tank vessel, **characterized in that** active agents in the form of aldehydes, ketones, esters and/or alcohols or natural, oily essences or perfume-like substances that react with and/or mask said foul-smelling substances and thereby reduce the annoyance due to the foul smell, are introduced together with the inert gas.

2. Process according to claim 1, **characterized in that** the tank vessel contains 500 to 15,000 to of heavy oil and/or bitumen, whereby the gas space above the liquid level accounts for 10 to 90% of the total volume.

3. Process according to claim 1, **characterized in that** nitrogen or water vapor is used as inert gas.

4. Process according to claim 1, **characterized in that** the foul-smelling substances contain hydrocarbons and sulfur compounds.

5. Process according to claim 1, **characterized in that** the active agents are aldehydes, ketones, esters and/or alcohols.

6. Process according to claim 1, **characterized in that** the active agents are injected into the inert gas flux as aqueous emulsion in the form of fine droplets with a particle size of less than 50 µm or as gas, and are introduced jointly with the inert gas flux into the gas space where they react with and/or mask the foul-smelling substances.

7. Process according to claim 6, **characterized in that** the active agents are injected as 0.5 to 25 wt-% aqueous emulsion through fine nozzles in the form of fine droplets with a particle size of 5 to 25 µm into the tube in which the inert gas flux is guided.

## Revendications

1. Procédé pour la réduction de substances nauséabondes dans des réservoirs destinés à du pétrole lourd ou à du bitume liquéfié, dans lesquels, au-dessus du niveau du liquide, se trouve un espace occupé par un gaz et renfermant les substances nauséabondes, un gaz inerte étant introduit dans l'espace réservé au gaz pour le rendre inerte vis-à-vis des explosions, avant qu'il ne ressorte par l'extrémité supérieure du réservoir, **caractérisé en ce qu'**on introduit, de manière conjointe avec le gaz inerte, des agents actifs sous la forme d'aldéhydes, de cétones, d'esters et/ou d'alcools ou d'huiles essentielles naturelles ou de substances du type des parfums, qui réagissent avec les substances nauséabondes respectivement qui masquent ces dernières, pour ainsi réduire la nuisance due à l'odeur.

2. Procédé selon la revendication 1, **caractérisé en ce que** le réservoir contient de 500 à 15.000 tonnes de pétrole lourd respectivement de bitume, l'espace occupé par le gaz au-dessus du niveau du liquide représentant de 10 à 90 % du volume total.

3. Procédé selon la revendication 1, **caractérisé en ce que** l'on introduit, à titre de gaz inerte, de l'azote ou de la vapeur d'eau.

4. Procédé selon la revendication 1, **caractérisé en ce que** les substances nauséabondes contiennent des hydrocarbures et des composés soufrés.

5. Procédé selon la revendication 1, **caractérisé en ce que** les agents actifs sont des aldéhydes, des cétones, des esters et/ou des alcools.

6. Procédé selon la revendication 1, **caractérisé en ce que** les agents actifs sont pressés dans le courant de gaz inerte sous la forme d'une émulsion aqueuse prenant la forme de fines gouttelettes dont la granulométrie est inférieure à 50 µm ou sous la forme d'un gaz, pour être introduits conjointement avec le courant de gaz inerte dans l'espace occupé par le gaz où ils réagissent avec les substances nauséabondes, respectivement masquent ces dernières.

7. Procédé selon la revendication 6, **caractérisé en ce que** les agents actifs sont pressés sous la forme d'une émulsion aqueuse de 0,5 à 25 % en poids via de fines buses sous la forme de fines gouttelettes dont la granulométrie s'élève de 5 à 25 µm pour pénétrer dans le tube dans lequel le courant de gaz inerte est guidé.
